# EUROPEAN PATENT SPECIFICATION

(11) **EP 0 767 374 B1**
(45) Date of publication and mention of the grant of the patent: **12.11.2003**
(21) Application number: 95115629.8
(22) Date of filing: 04.10.1995
(51) Int. Cl.: G01N 27/60, G01N 33/44, B29B 17/02

(54) **Plastics identification**
Identifizierung von Kunststoffen
Identification de plastiques

(43) Date of publication of application: 09.04.1997
(73) Proprietor: FORD MOTOR COMPANY LIMITED, Brentwood Essex (GB); FORD-WERKE AKTIENGESELLSCHAFT, 50735 Köln (DE); FORD FRANCE S.A., 92506 Rueil-Malmaison Cédex (FR); FORD MOTOR COMPANY, Dearborn, MI 48121 (US)
(72) Inventor: Gentle, Derek, Danbury, Essex CM3 4EH (GB); Mucci, Peter Edmund Rueben, Durley, Southampton (GB); Amner, John Arthur, Rochford, Essex SS4 1SR (GB); Hearn, Graham Leslie, Southampton, Hampshire SO2 1SZ (GB)
(74) Representative: Messulam, Alec Moses

(56) References cited:
- WO-A-94/17402
- KUNSTSTOFFBERATER, vol. 39, no. 6, June 1994 ISERNHAGEN DE, pages 28-33, XP 000460711 I. STAHL ET AL.
- EUROPEAN PLASTICS NEWS, vol. 20, no. 4, April 1993 CROYDON GB, page 10 XP 000413789 'Ford investigates plastics identification techniques'
- INTERNATIONAL POLYMER SCIENCE AND TECHNOLOGY, vol. 21, no. 8, 1994 SHREWSBURY GB, pages t/73-t/80, XP 000485150 A. K. BLEDZKI ET AL.

## Description

This invention relates to a device for identifying different plastics materials. The device is particularly (but not exclusively) suitable for use in separating plastics components into chemically similar groups, as a first stage in, for example, the recycling of the plastics materials. In this specifications, "plastics" includes both thermoplastic and thermoset materials, whether in the form of solid or foamed material and also includes natural and synthetic rubbers and any composite material which is a composition of any of these materials. The invention is also applicable to such materials in which additives (plastics or non plastics) have been included.

Our International Patent Application No. WO 94/17402, published 4 August 1994, describes and claims a method and an apparatus for identifying different plastics materials by rubbing different reference materials against the sample to be identified to create electrostatic charges which are then processed by a logic circuit to identify the nature of the sample.

The present invention provides an improved device for use in the method of that earlier invention.

According to the present invention, there is provided a device for identifying plastics materials, the device comprising at least one electrostatically chargeable detector head mounted on a housing in such a way that the head can be rubbed against a sample of unidentified plastics material, characterised in that at least one detector head has a surface of a plastics material which can be rubbed against the sample with the plastics material surface being itself secured to a support of an electrically conductive material which does not come into direct contact with the sample surface, the conductive support being connected to means for measuring the polarity of charge induced on the support, wherein such detector head is resiliently mounted in the housing so that when it is pressed against the sample, the resilient mounting is compressed.

The plastics material surface is preferably provided by a layer of plastics material which has a thickness of between 0.5 and 2 mm. 1 mm thickness is preferred. The electrically conductive material which supports the plastics surface can be a metal, for example brass.

Preferably the device is in the form of a portable, hand held unit which requires no external connections to external power or data handling equipment, although it is within the scope of the invention in its broadest terms for such connections to be made. Preferably the housing includes both the polarity measuring means and the power source for powering the measuring means and an indicator mounted on the housing.

The operator can therefore rub the detector head or heads against the sample and then see from the indicator on the housing the identity of the sample being tested.

The head or heads may be fixed on the housing so that rubbing of the heads against the sample is carried out by making a relative movement between the device and the sample. Alternatively, however, the sample may include a motor to rotate the detector head or heads against the sample, or there may be a mechanical drive which causes the head or heads to be moved against the sample when an arm or cover at the end of the device housing is displaced by pushing the device against the sample.

In one embodiment there is only one detector head, and this embodiment is useful in distinguishing between two different plastics materials. For example, if a quantity of material to be recycled is known to be either of type A or of type B, then a single detector head device can be used to separate the material into the two types.

In another preferred embodiment, there are three detector heads, one being of metal and two having plastics material surfaces, with different plastics materials on the two different heads.

The materials of the three detector heads may be brass, polymethylmethacrylate and polyvinylchloride. This combination of heads allows the identification and separation of polyamide-polypropylene-abs-polyethylene.

When the detector heads are mounted in the device so that the device has to be moved relative to the sample to take a reading, then the heads may be mounted resiliently, eg spring loaded, so that when the housing is pressed against the sample, contact between all the heads simultaneously is assured.

The invention will now be further described, by way of example, with reference to the accompanying drawings, in which:
Figure 1 is a perspective view of a device in accordance with the invention;
Figure 2 is a view on one end of the device of Figure 1;
Figure 3 is a view on the opposite end of the device of Figure 1;
Figure 4 is a perspective view of two of the detector heads from the device of Figure 1;
Figure 5 is a section through the two detector heads of Figure 4 showing the charge distribution;
Figure 6 is a schematic side view of the device of Figure 1; and
Figures 7, 8 and 9 show three alternative ways of producing a rubbing action between detector heads and a sample surface.

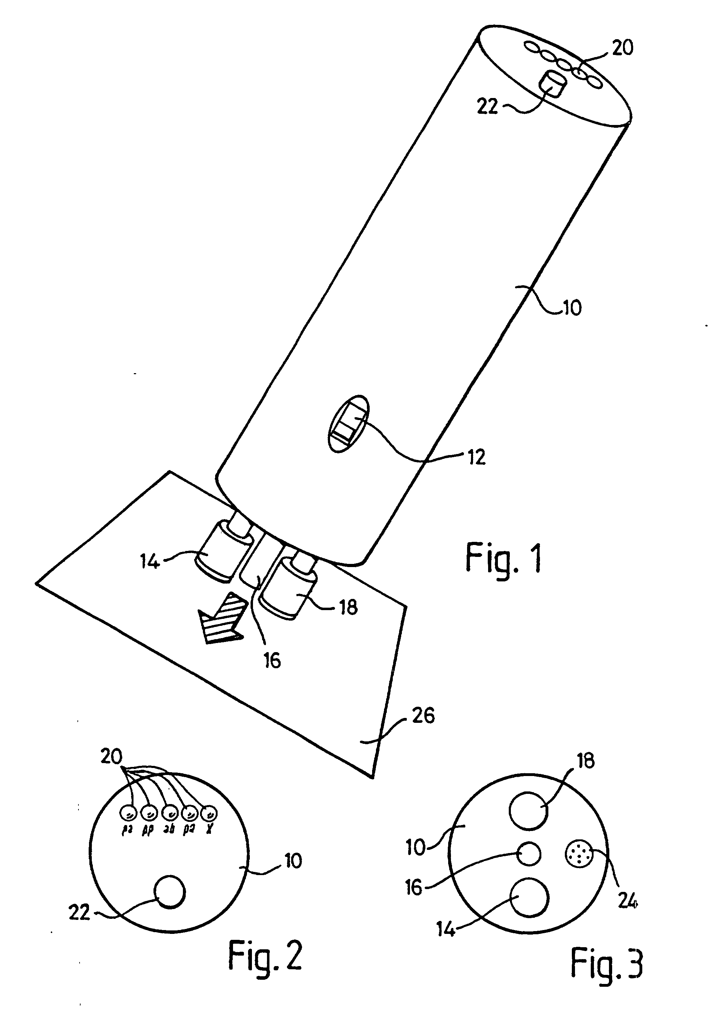

The device shown in Figure 1 has a housing 10 of cylindrical form of a size which can be easily held in the hand. Although a simple cylindrical form is shown in Figure 1, the housing could be shaped to make it comfortable to hold in the hand, in a known way.

The device has an ON/OFF switch 12 and three detector heads 14, 16, 18 at one end. At-the other end the housing has five indicator lamps 20 and a reset button 22. Figure 2 shows the opposite end of the device and it will be seen that there are five indicators 20. In the device shown, with three detector heads, it is possible to separately identify four different plastics types, so four of the lights relate to specific plastics types and in use an appropriate one of the lights will light up in accordance with the plastic type detected. The fifth light indicates a failed reading and tells the operator that the device must be reapplied to the sample surface and another reading taken.

Figure 1 shows the sample surface against which the device is rubbed at 26.

The view of the detector end of the device in Figure 3 shows, in addition to the three detector heads 14, 16, 18, a data connection outlet 24 to which electrical connections can be made to take off data from the device to an external display or store. Provision may be made, alternately or additionally, for data to be transferred from the device using radio signals.

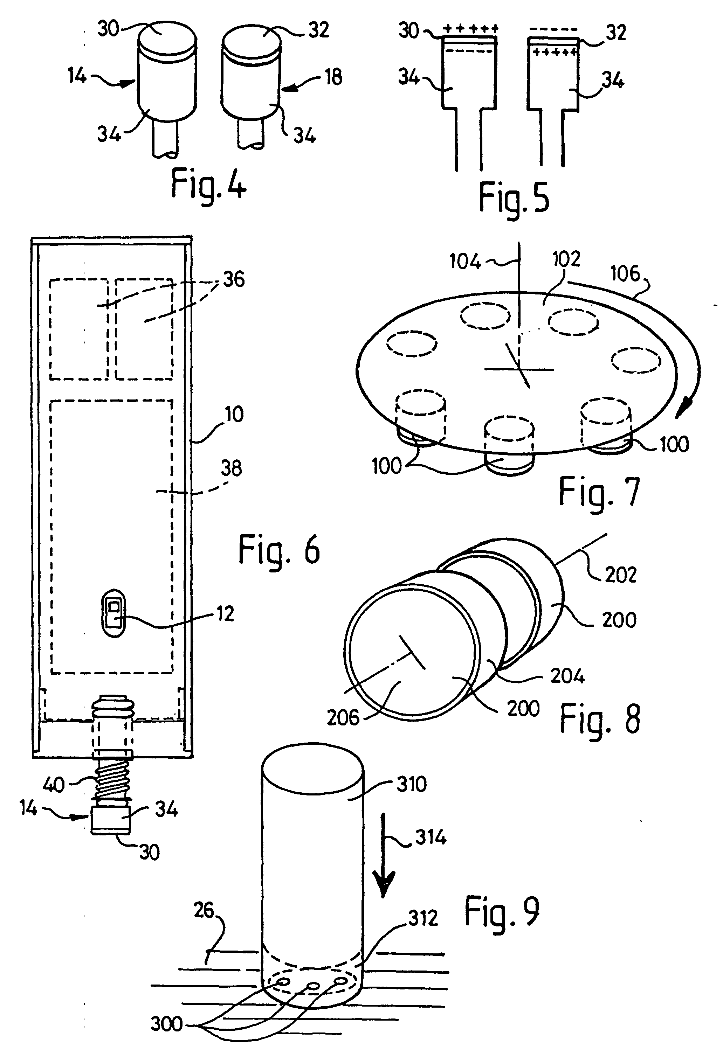

The detector head 16 is a simple metal probe which is arranged so that it can be rubbed against the sample surface. The detector heads 14 and 18 are shown in more detail in Figure 4. Each head comprises a plastics disc 30, 32 mounted, for example by adhesive, on the end of a conductive cylinder 34 which is brass in this example. The plastics discs 30, 32 which may suitably be 1 mm thick and 10 mm in diameter are of different plastics materials. For example, the disc 30 may be of polymethylmethacrylate (PMMA) and the disc 32 can be of polyvinylchloride (PVC).

At least the heads 14 and 18 could be removable from the housing so that they can be readily exchanged for alternative heads with different plastics material surfaces 32, 34 to enable the device to be used to separate different categories of plastics.

When the heads are brought into frictional contact with the surface of the sample 26 by rubbing one against the other, electrostatic charge is generated on the face of each plastic disc 30, 32 by triboelectrification. The magnitude and polarity of the electrostatic charge generated in this way will vary depending on the material of the sample surface.

When the detector heads are moved away from the sample, the charge generated on the surface of the plastic disc is retained and the disc is electrically polarised as shown in Figure 5, by an induced charge on the brass cylinder 34. The charge induced in the cylinder 34 is detected by electrometer circuitry within the housing 10.

In use therefore, the device is switched on at the switch 12 and is then rubbed against the surface of the sample 26 with all the three heads 14, 16, 18 being in contact with the sample surface. What is important is that there be relative movement between the heads and the surface because it is this relative movement which creates, by the phenomenon of triboelectrification, an electrostatic charge on the detector heads. The nature of the charges produced on the three heads will be analysed by electrometer circuitry, and the circuitry will then illuminate the appropriate indicator 20.

Prior to taking another reading, the reset button 22 will be pressed. It may be possible to have an automatic reset by placing the button 22, or equivalent, in a position where it is pressed automatically by the action of pushing the housing against the sample surface 26.

Figure 6 illustrates schematically the components within the housing 10. Two batteries 36 are provided to power the indicators 20 and the electrometer circuitry, including the relays within this circuitry, indicated at 38. The circuitry 38 analyses the charges from each of the detector heads 14, 16, 18 and produces an output indicating the identity of the sample surface 26, in the manner described in our International Patent Application No. WO 94/17402.

Figure 6 also shows how the head 14 is spring-mounted at the bottom of the housing 10. A helical compression spring 40 surrounds a shank of the brass cylinder 34 and operates between a flange on the cylinder and a flange on the head so that when the device is pressed against a sample surface 26, the spring is compressed and this ensures that whatever the irregularities of the surface of the sample 26, contact is maintained between the sample and the heads.

Although a helical compression spring is shown in Figure 6, it will be understood that many alternative ways of providing the biasing action can be utilised. In order to maintain the correct orientation between the surfaces of the sample and of the detector heads, the detector heads could be connected to the housing by universal joints.

The embodiment described above has three detector heads. The invention can however equally be applied to a device which has only one detector head. In this case, the one head will be constructed along the same principles as the head 14 or 18. With only one head, less complex circuitry can be used and a lesser number of indicator can be used. The housing of such a device can therefore be significantly reduced in size, such that it takes on the general form of a pen or other writing instrument.

As made clear above, it is essential that there be relative movement between the detector head or heads and the sample surface 26. In the simplest embodiment, the detector heads are stationary in respect to the housing 10 (although they may be resiliently mounted as just described) and the relative movement is produced by moving the entire housing 10 relative to the sample surface 26. In the three alternative embodiments shown in Figures 7, 8 and 9 the housing 10 will be held stationary and only the detector heads will move relative to the sample surface.

In Figure 7, a large number (seven are shown) of detector heads 100 are mounted on a plate 102. The plate is mounted for rotation about an axis 104 as indicated by an arrow 106. In this case, the housing 10 would incorporate an electric motor to rotate the plate 102 when the heads are in contact with the sample surface, so that a friction induced charge appears on the detector heads 100.

By using a larger number of detector heads of different materials, it becomes possible to identify a larger number of materials and generally if the number of detector heads is n, then the number of materials that can be distinguished from one another is n + 1.

Figure 8 shows an arrangement which uses annular instead of cylindrical detector heads. Two detector heads 200 are shown mounted for rotation about their cylinder axes on an axis 202. The heads 200 have an outer surface with a thin plastics layer 204 backed by a solid brass body 206. The plastics layer 204 and the body 206 work together in the same way as the disc 30, 32 and the support 34 of Figures 4 and 5. This embodiment also requires a motor in the housing 10 to produce rotation of the detector heads 200 when they come into contact with a sample surface 26.

In Figure 9, three detector heads 300 will be mounted on a plate similar to the plate 102 of Figure 7. The housing 310 has a skirt 312 which is biased in a downward direction in the orientation of Figure 9. When the device is offered up to a sample surface 26, downward pressure on the housing 310 causes the skirt 312 to be displaced relative to the housing, and this displacement, through a suitable mechanical linkage, causes a corresponding angular displacement of the plate carrying the detector heads 300 to produce the required relative motion between the detector heads 300 and the sample surface 26.

Clearly many different mechanical arrangements could produce this relative motion resulting from downward pressure as indicated by arrow 314 on the housing 310.

If the detector heads become dirty during use, they may either be periodically replaced or they may be regularly cleaned. The device may have a cap or a fixed 'docking station' in which the device is placed when not in use, and this can include some arrangement for cleaning the heads when the cap is fitted or when the device is placed in the docking station. Such a station could also include a battery charger to recharge the batteries 36 when the device is not in use. It may also be desirable to deionise the head or heads between readings. This can be done by ejecting a small quantity of alcohol onto the head surface which will have the effect of removing any remaining charge on the head before evaporating.

When the device is out of use, it may be convenient also to carry out a calibration or reference check to ensure continued accurate operation, and the equipment to do this can be included in a docking station.

## Claims

1. A device for identifying plastics materials, the device comprising at least one electrically isolated and electrostatically chargeable detector head (14, 18) mounted on a housing (10) in such a way that the head can be rubbed against a single sample of unidentified plastics material, **characterised in that** at least one detector head (14, 18) has a surface (30, 32) of a plastics material which can be rubbed against the sample with the plastics material surface (30, 32) being itself secured to a support (34) of an electrically conductive material which does not come into direct contact with the sample surface, the conductive support (34) being electrically connected to means for measuring the polarity of charge induced on the support, wherein such detector head (14, 18) is resiliently mounted in the housing so that when it is pressed against the sample, the resilient mounting (40) is compressed,

2. A device as claimed in Claim 1 and having a plurality of detector heads arranged so that they can be rubbed simultaneously against a single sample of unidentified plastics material.

3. A device as claimed in Claim 2, wherein one of the heads is of metal and has no plastics material surface.

4. A device as claimed in Claim 2 or Claim 3, wherein there are three detector heads, one of metal and two with plastics material surfaces, the surfaces being of different plastics materials.

5. A device as claimed in Claim 4, wherein the different plastics materials are polymethylmethacrylate and polyvinyl chloride.

6. A device as claimed in any one of Claims 2 to 5 including a plurality of resiliently mounted detector heads, wherein each resilient mounting (40) is independent of the other resilient mountings.

7. A device as claimed in any preceding claim, wherein rubbing of the detector heads against the sample is carried out by making a relative movement between the device and the sample.

8. A device as claimed in any preceding claim, wherein the plastics material surface is provided by a layer of the material with a thickness of between 0.5 and 2 mm.

9. A device as claimed in any preceding claim, wherein the electrically conductive material is brass.

10. A device as claimed in any preceding claim, wherein the polarity measuring means is incorporated in the housing on which the heads are mounted.

11. , A device as claimed in Claim 10, wherein the housing incorporates means (20) for indicating the identity of the sample of unidentified plastics material.

12. A device as claimed in Claim 11, wherein the indicating means is a visual display.

13. A device as claimed in Claim 11 or Claim 12, wherein the indicating means is a series of individually illuminable, labelled indicators.

14. A device as claimed in any one of Claims 10 to 13, wherein the housing includes a power source for powering the measuring means and the indicating means.

15. A device as claimed in any one of Claims 10 to 14, wherein the housing is adapted to be held in the hand,

16. A device as claimed in any one of the preceding claims wherein the detector heads are removable from the housing for exchange with alternative detector heads,

## Patentansprüche

1. Eine Vorrichtung zur Identifizierung von Kunststoffmaterialien, wobei die Vorrichtung mindestens einen elektrisch isolierten und elektrostatisch aufladbaren Detektorkopf (14, 18) umfaßt, der in solcher Art und Weise an einem Gehäuse (10) montiert ist daß der Kopf gegen eine einzelne Probe von nicht identifiziertem Plastikmaterial gerieben werden kann; **dadurch gekennzeichnet daß** mindestens ein Detektorkopf (14, 18) eine Oberfläche (30, 32) aus einem Plastikmaterial aufweist, welches mit der Plastikmaterial-Oberfläche (30, 32) - die selbst an einem Träger (34) aus einem elektrisch leitfähigen Material befestigt ist, welches mit der Probenoberfläche nicht in direkten Kontakt kommt - gegen die Probe gerieben werden kann; wobei der leitfähige Träger (34) elektrisch an Vorrichtungen zur Messung der Polarität jener auf dem Träger induzierten Ladung angeschlossen ist, und worin ein derartiger Detektorkopf (14, 18) federnd in dem Gehäuse montiert ist, so daß die federnde Halterung (40) zusammengedrückt wird wenn sie gegen die Probe gepreßt wird.

2. Eine Vorrichtung gemäß Anspruch 1 und eine Mehrzahl von Detektorköpfen so arrangiert aufweisend, daß sie gleichzeitig gegen eine einzige Probe von nicht identifiziertem Plastikmaterial gerieben werden können.

3. Eine Vorrichtung gemäß Anspruch 2, in der einer der Köpfe aus Metall besteht und keine Plastikmaterial-Oberfläche besitzt.

4. Eine Vorrichtung gemäß Anspruch 2 oder Anspruch 3, in der es drei Detektorköpfe gibt, einen aus Metall und zwei mit Plastikmaterial-Oberflächen; wobei die Oberflächen aus verschiedenen Plastikmaterialien bestehen.

5. Eine Vorrichtung gemäß Anspruch 4, in der die verschiedenen Plastikmaterialien Polymethylmethacrylat und Polyvinylchlorid sind.

6. Eine Vorrichtung gemäß einem der Ansprüche 2 bis 5, einschließlich einer Mehrzahl federnd montierter Detektorköpfe, worin jede federnde Aufnahme (40) unabhängig von den anderen federnden Halterungen ist.

7. Eine Vorrichtung gemäß einem der vorstehenden Ansprüche, in der ein Reiben der Detektorköpfe gegen die Probe ausgeführt wird indem man eine relative Bewegung zwischen der Vorrichtung und der Probe verrichtet.

8. Eine Vorrichtung gemäß einem der vorstehenden Ansprüche, in der die Plastikmaterial-Oberfläche durch eine Schicht des Materials mit einer Stärke zwischen 0,5 und 2 mm bereitgestellt wird.

9. Eine Vorrichtung gemäß einem der vorstehenden Ansprüche, in dem das elektrisch leitfähige Material Messing ist.

10. Eine Vorrichtung gemäß einem der vorstehenden Ansprüche, in dem jene die Polarität messende Vorrichtung in das Gehäuse integriert ist, auf welchem die Köpfe montiert sind.

11. Eine Vorrichtung gemäß Anspruch 10, in der das Gehäuse Vorrichtungen (20) einschließt, um die Identität der Probe von nicht identifiziertem Plastikmaterial anzuzeigen.

12. Eine Vorrichtung gemäß Anspruch 11, in der die Anzeigevorrichtung eine visuelle Anzeige ist.

13. Eine Vorrichtung gemäß Anspruch 11 oder Anspruch 12, in der die Anzeigevorrichtungen eine Reihe von einzeln beleuchtbaren, beschrifteten Anzeigen ist.

14. Eine Vorrichtung gemäß einem Ansprüche 10 bis 13, in der das Gehäuse eine Energiequelle zum Betrieb der Meßvorrichtungen und der Anzeigevorrichtungen einschließt.

15. Eine Vorrichtung gemäß einem der Ansprüche 10 bis 14, in der das Gehäuse angepaßt ist um in der Hand gehalten zu werden.

16. Eine Vorrichtung gemäß irgendeinem der vorstehenden Ansprüche, in dem die Detektorköpfe zum Austausch mit alternativen Detektorköpfen von dem Gehäuse entfernbar sind.

## Revendications

1. Dispositif destiné à identifier des matières plastiques, le dispositif comprenant au moins une tête de détection électriquement isolée et pouvant être chargée de façon électrostatique (14, 18) montée sur un boîtier (10) de telle manière que la tête puisse être frottée contre un seul échantillon de matière plastique non identifiée, **caractérisé en ce qu'**au moins une tête de détection (14, 18) présente une surface (30, 32) d'une matière plastique qui peut être frottée contre l'échantillon, la surface de matière plastique (30, 32) étant elle-même fixée à un support (34) d'un matériau électroconducteur qui ne vient pas en contact direct avec la surface de l'échantillon, le support conducteur (34) étant électriquement relié à un moyen destiné à mesurer la polarité de la charge induite sur le support, dans lequel une telle tête de détection (14, 18) est montée élastiquement dans le boîtier de sorte que lorsqu'elle est pressée contre l'échantillon, le montage élastique (40) est comprimé.

2. Dispositif selon la revendication 1 et comprenant une pluralité de têtes de détection disposées de sorte qu'elles puissent être frottées simultanément contre un seul échantillon de matière plastique non identifiée.

3. Dispositif selon la revendication 2, dans lequel l'une des têtes est en métal et ne présente aucune surface de matière plastique.

4. Dispositif selon la revendication 2 ou la revendication 3, dans lequel il existe trois têtes de détection, l'une de métal et deux avec des surfaces de matière plastique, les surfaces étant de matières plastiques différentes.

5. Dispositif selon la revendication 4, dans lequel les matières plastiques sont du polyméthylméthacrylate et du polychlorure de vinyle.

6. Dispositif selon l'une quelconque des revendications 2 à 5, comprenant une pluralité de têtes de détection montées élastiquement, dans lequel chaque montage élastique (40) est indépendant des autres montages élastiques.

7. Dispositif selon l'une quelconque des revendications précédentes, dans lequel le frottement des têtes de détection contre l'échantillon est exécuté en effectuant un déplacement relatif entre le dispositif et l'échantillon.

8. Dispositif selon l'une quelconque des revendications précédentes, dans lequel la surface de matière plastique est réalisée par une couche de la matière présentant une épaisseur entre 0,5 et 2 mm.

9. Dispositif selon l'une quelconque des revendications précédentes, dans lequel le matériau électroconducteur est du laiton.

10. Dispositif selon l'une quelconque des revendications précédentes, dans lequel le moyen de mesure de polarité est incorporé dans le boîtier sur lequel les têtes sont montées.

11. Dispositif selon la revendication 10, dans lequel le boîtier incorpore un moyen (20) destiné à indiquer l'identité de l'échantillon de matière plastique non identifiée.

12. Dispositif selon la revendication 11, dans lequel le moyen d'indication est un affichage visuel.

13. Dispositif selon la revendication 11 ou la revendication 12, dans lequel le moyen d'indication est une série d'indicateurs marqués pouvant être illuminés individuellement.

14. Dispositif selon l'une quelconque des revendications 10 à 13, dans lequel le boîtier comprend une source d'alimentation destinée à alimenter le moyen de mesure et le moyen d'indication.

15. Dispositif selon l'une quelconque des revendications 10 à 14, dans lequel le boîtier est conçu pour être tenu à la main.

16. Dispositif selon l'une quelconque des revendications précédentes, dans lequel les têtes de détection sont séparables du boîtier en vue d'un échange par d'autres têtes de détection.
